Europäisches Patentamt

European Patent Office

Office européen des brevets

(11) Publication number: **0 363 819 B1**

# EUROPEAN PATENT SPECIFICATION

(45) Date of publication of patent specification: **29.12.93**  (51) Int. Cl.⁵: **A01H 1/02**

(21) Application number: **89118475.6**

(22) Date of filing: **05.10.89**

(54) Method for producing male-sterile tomato plants.

(30) Priority: **08.10.88 JP 254297/88**

(43) Date of publication of application:
**18.04.90 Bulletin 90/16**

(45) Publication of the grant of the patent:
**29.12.93 Bulletin 93/52**

(84) Designated Contracting States:
**AT BE CH DE ES FR GB GR IT LI LU NL SE**

(56) References cited:
**EP-A- 0 214 601
EP-A- 0 235 697
EP-A- 0 262 666
DE-A- 2 842 179
US-A- 4 734 369**

**Theoretical and Applied Genetics vol. 58,
1980, pages 121-127, D. Aviv & E.Galun:
"Restoration of fertility in cytoplasmicmale
sterile (CMS) Nicotiana sylvestris by fusion
with X-radiated N. tabacom protoplasts"**

**Science vol. 219, 11 February 1983, pages
683 - 688; J.F.Shepard et al: "Genetic transfer
in plants through interspecific protoplast fu-
sion"**

(73) Proprietor: **NICHIREI CORPORATION
6-19-20 Tukiji,
Chuo-ku
Tokyo 104(JP)**

(72) Inventor: **Melchers, Georg
Correns Strasse 45
D-7400 Tübingen(DE)**
Inventor: **Mohri, Yoshiharu
1-7-10-301, Kita-machi
Hoya Tokyo(JP)**
Inventor: **Watanabe, Kazuo
4-37--10, Nakaarai
Tokorozawa Saitama(JP)**
Inventor: **Wakabayasi, Susumu
2-1-3-610 Namiki
Tokorozawa Saitama(JP)**
Inventor: **Harada, Kazuhiko
3-19-88-202, Kitanaka
Tokorozawa Saitama(JP)**
Inventor: **Sakai, Miho
2-6-26-302, Asahigaoka
Asaka Saitama(JP)**
Inventor: **Sato, Eko
603-1-15-402, Bushi
Iruma Saitama(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.6/3.3.1)

P. Röhlich & E. Bacsy (ed): "Tissueculture and reticuloendothelial system"; Proceedings 1983, 1984 VNU Science Press, Utrecht pages 499-513 G. Melchers: "Topatoes and pomatoes somatic hybrids between tomatoes ane potatoes"

P.V, Annurati (ed): "Handbook of plant cell culture Vol. 3 Crop Species", 1984, Macmillan, New York Ch. 10 pages 247-289 S.A. kut et al:"Tomato"

(74) Representative: **Patentanwälte Ruff, Beier, Schöndorf und Mütschele**
**Willy-Brandt-Strasse 28**
**D-70173 Stuttgart (DE)**

**Description**

Background of the Invention

The present invention relates to the production of tomato plants that are male-sterile. In the present context, a plant is deemed "male-sterile" if it produces no pollen or at least no germinateable pollen, and is therefore virtually incapable of self-fertilization, but can be a female parent in a cross with a male-fertile plant.

Open-pollinated tomatoes which are currently grown have been surpassed by a number of $F_1$ hybrid tomato cultivars in key aspects like yield, uniformity of fruit production and net productivity. But because tomato is an in-breeding crop, laborious manual emasculation and pollination have been prerequisites heretofore to the exploitation of hybrid vigour in commercial hybrid fruit production.

One alternative to manipulation of the female parent, thereby preventing self-fertilization, is to use male-sterile or other sterile mutants as female parents. Some 55 genes that effect sterility (genic male sterility or g-mst) are known in tomato, Lycopersicon esculentum Miller, and g-mst tomato lines have been used on a limited basis for commercial hybrid fruit and seed production in Israel, Bulgaria, France and the United States.

But difficulties encountered in producing a field population made up primarily of g-mst seed parent plants ("homogenous stands") have hindered wider use of genic male sterility in tomato hybridization. In addition, the introgression of g-mst determinants (ms) into a desired background usually entails several generations of crossing and selection before male-sterile plants are obtained which are otherwise identical to those of the desired cultivar. For these reasons, the production of $F_1$ tomato seeds has hitherto been labor-intensive and time-consuming, and production costs have been high.

Another alternative, at least in theory, would be the exploitation of gene-cytoplasmic male sterility in the context of hybrid tomato production. In general terms, nuclear ms genes are cytoplasm-insensitive, while the action of nuclear determinants (fr genes) affecting male fertility is cytoplasm-sensitive. Thus, fr genes act only in a certain cytoplasm type (the S-cytoplasm), containing specific c genes, to produce a "gene-cytoplasmic" male sterility (gc-mst).

According to Japanese patent application No. 63-500001, for example, male-sterile characteristics can be transferred into a maintainer variety of carrot (Daucus carota) by combining the nucleus of the maintainer variety with the enucleated cytoplasm of a gc-mst carrot variety. Neither this strategy nor any other based on gene-cytoplasmic male sterility is feasible in tomato, however, because no useful S-cytoplasm has been found in L. esculentum. EP-A-214 601 describes a process for perpetuating male sterile plants, for example of the Solanaceae or Nicotiana tabacum species, by male sterile nucleus and male fertile cytoplasm protoplast fusion, regeneration of the obtained cybrid to a male fertile plant, and crossing it with a cytoplasmic male sterile plant.

Summary of the Invention

It is therefore an object of the present invention to provide a method for producing male-sterile tomato plants that is practical to carry out on a commercial level and can be effected efficiently in one step, i.e., within a relatively short time without crossing or backcrossing.

It is also an object of the present invention to provide the means for conducting commercial-scale production of hybrid tomato fruit or seed without recourse to the expensive, labor-intensive techniques heretofore available.

In accomplishing these objects, there has been provided, in accordance with the present invention, a method for the production of male-sterile tomato plants, comprising the steps of treating tomato protoplasts with a cytoplasmic inactivator to produce tomato protoplasts that contain inactivated cytoplasmic elements; further comprising the step of producing Solanum protoplasts that contain inactivated nuclear elements, said step comprising an irradiation either of a Solanum plant or of Solanum protoplasts with a sterilizing dose of gamma-ray or x-ray radiation; followed by the steps of fusing said tomato protoplasts that contain inactivated cytoplasmic genetic material with said Solanum protoplasts that contain inactivated nuclear genetic material, to obtain a plurality of fusion products; and regenerating at least one fusion product of said plurality into a whole, male-sterile tomato plant.

In accordance with the present invention, a male-sterile tomato plant is provided that is the product of the above-described method. Also provided is a homogeneous stand of male-sterile tomato plants, which was heretofore inaccessible in practice.

3

Other objects, features and advantages of the present invention will become apparent from the following detailed description. It should be understood, however, that the detailed description and the specific examples, while indicating preferred embodiments of the invention, are given by way of illustration only, since various changes and modifications within the spirit and scope of the invention will become apparent to those skilled in the art from this detailed description.

Detailed Description of Preferred Embodiments

It has been discovered that an incompatibility between nuclear genes and cytoplasmic genes can be employed to advantage in a method for producing male-sterile tomato plants, which method involves introducing foreign cytoplasm, but not foreign nuclear genes, into the cells of any desired tomato cultivar. In particular, protoplasts of a tomato cultivar which is to be made male-sterile, according to the present invention, are treated with a cytoplasmic inhibitor, in order to inactivate cytoplasmic factors therein, and are then fused with protoplasts, obtained from a plant of a Solanum species, wherein nuclear genes have been inactivated. Total sterility, as has been reported from somatic hybrids regenerated from heterokaryons produced via fusing untreated protoplasts of tomato and potato (Solanum tuberosum), see Melchers et al.,Carlsberg Res. Commun. 43: 203-18 (1978); Shepard et al., Science 219: 683-89 (1983), is avoided by this approach, while a desirable form of partial sterility (male sterility) is achieved.

The distinction between the two sources for protoplasts used in the present invention is well-understood taxonomically. As indicated above, the commercial tomato belongs to a species, of the family Solanaceae, that is most frequently referred to as Lycopersicon esculentum Miller, although alternative names have appeared (Lycopersicon lycopersicum and Solanum lycopersicum L., for example). The family Solanaceae is mainly divided into two sub-families, the Solanoideae and the Cestroideae, the former of which is further subdivided into tribes. Lycopersicon belongs to the largest of these, the tribe Solaneae, which also includes the genus Solanum.

Members of the family Solanaceae share a general flower structure, characterized by petals that are fused together, at least at the base, and by stamen filaments that are fused to the petals so that the stamens appear to be arising from the corolla. The two genera, Lycopersicon and Solanum, are traditionally separated on the basis of the unique anther morphology usually found in Lycopersicon species, including the commercial tomato. The flower of Lycopersicon normally has five anthers (some varieties of the crop species L. esculentum have six), and the stamens are joined together to form a flask-shaped anther cone which is characteristic of the genus. The "neck" of the cone is made up of the elongated sterile tip of each anther, and it is this characteristic that has been used widely to distinguish Lycopersicon from Solanum. Also associated with this characteristic is an unusual pattern of anther dehiscence found in Lycopersicon species, whereby the anthers split laterally, in contrast to the terminally dehiscent anthers found in Solanum.

The biosystematic distance between L. esculentum and the Solanum species employed as a protoplast source, according to the present invention, is not crucial; that is, the desired male sterility can be achieved, with reasonable reproducibility, whenever cytoplasm-inactivated tomato protoplasts are fused with nuclear-inactivated protoplasts derived from a Solanum plant. Preferred as sources for protoplasts employed in the present invention are potato plants (S. tuberosum) and plants of a Solanum species that is deemed "potato-like" by virtue of possessing an underground stem or tuber (storage rhizome) with shortened internodes. Pursuant to this criterion many Solanum species, such as S. berthaultii Hawkes, S. chacoense, S. tarijense Hawkes, S. kurtzianum, S. demissum Lindl. and S. polyadenium Greenm., qualify as potato-like.

Exemplary of the potato-like Solanum series is the tetraploid species S. acaule Hawkes. Solanum acaule grows as a rosette at low temperatures, elongates at temperatures in the range of 20° to 30°C, and has fruit that are high-round and green in color. The species can also propagate from tubers and, in this respect, resembles S. tuberosum. The petals of S. acaule are white or purple, and its flowers, while much smaller, have a morphology resembling that of the potato. The pronounced frost resistance of S. acaule identifies the species as an attractive potential source for cold resistance in tomato breeding program. See Melchers, "Topatoes and Pomatoes, Somatic Hybrids Between Tomatoes and Potatoes," in TISSUE CULTURE AND RES 499, 505 (Akademiai Kiado, Budapest 1984). Solanum acaule is self-fertile but it does not cross sexually with a fact that has made the production of S. acaule/L. esculentum hybrids heretofore impossible.

Solanum acaule Hawkes is indigenous to the higher altitudes of the Andes, where plants of this species are readily obtainable. In addition, S. acaule has been cultured for many years at the Max-Planck Institut fur Zuechtungsforschung (Cologne, FGR) and, more recently, at the Max-Planck Institut fur Biologie (Tuebingen, FGR). Propagating material of the species is available from both of these sources, as well as from Centro Internacional de la Papa (P.O. Box 5969, 100 Lima, Peru), a public institution and an international center for potato research.

Protoplasts for use in the present invention can be produced by conventional enzymatic techniques. There are a number of commercially available enzymes, such as "Cellulase Onozuka R-10" and the pectinase "Macerozyme R-10," both products of Yakult Honsha Co. (Tokyo) that are suitable for use in this context.

As described, for example, by Eriksson, "Protoplast Isolation and Culture," in PLANT PROTOPLASTS 1-20 (CRC Press 1983) (hereafter "Eriksson"), the contents of which are hereby incorporated by reference, the enzymatic isolation of protoplasts can be performed in two different ways: the two-step (or sequential method) and the one-step method. In the two-step method, the tissue is first treated with a macerozyme or pectinase which separates the cells by degrading the middle lamella. The freed cells are then treated with cellulase, which releases the protoplasts. In general, the cells are exposed to the different enzymes for shorter periods than are used in the one-step method. In the one-step method the tissue is subjected to a mixture of enzymes, including macerozyme and cellulase. This method generally results in higher yields from leaf tissues, since mesophyll (palisade and spongeous) cells release protoplasts, and is also less labor intensive.

Since protoplasts are negatively charged and will not spontaneously fuse at usable frequencies, fusion of the protoplasts has to be induced. Preferred approaches in this regard entail the use of chemical fusion agents such as high calcium and high pH, see Keller and Melchers, Z. Naturforsch. 28c: 737-41 (1973), and polyethylene glycol (PEG), which is usually employed as a solution of PEG 1540, 4000 or 6000. Electrical methods can also be used to induce protoplast fusion, for example, via the electric field pulse technique disclosed by Vienken et al., Physiol. Plant. 53: 64 (1981).

Cell fusion between tomato and Solanum protoplasts can thus be effected in a conventional manner. From calli developed from the resulting fusion products, whole plants can be regenerated pursuant to available methodology as described, for example, by Shahin, Theor. Appl. Genet. 69: 235-40 (1985) (hereafter "the Shahin article"), Melchers and Labib, Molec. Gen. Genet. 135: 277-94 (1974), and Kao et al., Planta 120: 215-27 (1974), the respective contents of which are hereby incorporated by reference. In general, about 20% to 30% of the calli obtained in this manner are regenerable into plants. Calli from which can be regenerated male-sterile tomato plants within the present invention are obtained at a frequency, typically in the range of $10^{-1}$ and $10^{-3}$, that is readily accommodated with available callus selection/regeneration techniques.

Pursuant to the present invention, tomato protoplasts are treated in such a way as to inactivate genetic material contained in the cytoplasm. Preferably, this is accomplished by exposing the tomato protoplasts to a compound (hereafter "cytoplasmic inactivator"), like iodoacetic acid or Rhodamine 6-G, that blocks replication of, or otherwise disrupts, mitochondrial DNA. A preferred cytoplasmic inactivator in this context is iodoacetate or one of its derivatives, such as iodoacetamide. Cytoplasmic inactivation can be carried out, in accordance with the present invention, by suspending tomato protoplasts temporarily in an aqueous solution containing an amount of the cytoplasmic inactivator that has been determined empirically to be effective in disrupting cytoplasmic genetic material.

Thus, the specific concentration of cytoplasmic inactivator which is optimal can be readily ascertained with regard, for example, to the strain and physiological condition of the source from which the tomato protoplasts are obtained. For the tomato protoplasts used in the examples below, suitable concentrations of the cytoplasmic inactivator of choice (iodoacetamide) fell in the range of about 5 to 30 mM.

Solanum protoplasts which are to be fused with tomato protoplasts, according to the present invention, should be treated so that transmission of nuclear genes to the fusion products is prevented. This nuclear inactivation is preferably accomplished by irradiating the donor plant (before isolation of protoplasts) or the protoplasts themselves, after isolation, with a suitable dose of γ- or x-rays. As with the cytoplasmic inactivator, the optimal dosage of radiation for a given situation can be determined by routine experimentation. For S. acaule it has been found that a dose of γ- or x-rays within the range of 10 to 200 krad is suitable.

In the present invention, cytoplasm-inactivated tomato protoplasts and Solanum protoplasts containing an inactivated nuclear-genetic complement are subjected to cell fusion in order to give rise to an altered cooperation between nucleus and cytoplasm, without producing significant adverse mutagenic effects on the nuclear genes of the tomato plants, thereby producing male-sterile tomato plants. The anthers and pollen of the male-sterile tomato plants of the present invention typically differ in morphology from the original ("parent") male- fertile plants as follows:

(A) The male-sterile plant may have normal anthers, containing pollen grains that all stain with acetocarmine (an indication of viability) but do not germinate.

(B) The male-sterile plant may have normal anthers but many of its pollen grains are shrunken and do not germinate.

(C) The male-sterile plant may have shrunken anthers and no pollen.

(D) The male-sterile plant may have normal anthers but either (i) significantly fewer than 100% of its pollen grains stain with acetocarmine or (ii) some of the pollen grains germinate when placed on artificial pollen-germination medium.

Type (D) plants are potential sources of a suitable maintainer (B-line) genotype for use in sustaining an otherwise identical but male-sterile tomato line produced according to the present invention. In any event, such plants are easily differentiated from type (A), type (B) and type (D) plants, based on the criteria of pollen appearance and stainability discussed above.

Except for the anthers and pollen, no morphological differences are readily detected between the aforementioned types of male-sterile plants of the present invention and the parent plants. Plants regenerated from fusion products, according to the present invention, thus differ from the fertile parent plants in that the pollen of the former do not germinate on the stigmas of fertile tomato plants under normal tomato-culture conditions.

The present invention is further described by reference to the following, illustrative examples. The examples show how novel hybrid tomato cultivars were obtained, as described above, using plants which represent five tomato cultivars, respectively, and an exemplary Solanum species.

Example 1.

The inbred tomato cultivar "Sekai-ichi" was selected for conversion to male-sterility. According to the ENCYCLOPEDIA OF HORTICULTURE, Vol. 6 (Seibundo Shinkosha, 1970), Prof. Taniguchi of Tokyo Higher Agricultural College characterized "Sekai-ichi" in 1933 as a selection from cv. "Ponderosa" or a hybrid obtained via a cross involving cv. "Beefheart." "Sekai-ichi" grows vigorously and abundantly, and is a medium or slow grower. It grows well in places where "Ponderosa" also grows well but is harvested slightly earlier. The fruit of "Sekai-ichi" is pink, flat and larger-than-average, with pointed tops, deep hollows and fimbriae extending to the back.

Tomato seeds from "Sekai-ichi" were aseptically germinated, and the cotyledons were dissected from the seedlings and placed in a modified version of the TSE-2 enzyme solution described by Shahin in CELL CULTURE AND SOMATIC CELL GENETICS OF PLANTS, Vol. 1, page 375 (Vasil ed. Academic Press, 1984), to obtain protoplasts. The constituency of the enzyme solution was modified to contain 2% Cellulase Onozuka R-10 and 0.2% Macerozyme R-10.

The protoplasts thus produced were isolated and suspended in a solution containing 10 mM iodoacetamide, and were then allowed to stand at 4°C for 15 minutes to inactivate the cytoplasmic factors of the protoplasts.

Separately, Solanum acaule plants were aseptically propagated by cutting. The leaves were dissected and irradiated with $\gamma$- or x-rays to inactivate their nuclear genes. Subsequently, the leaves were treated with the modified TSE-2 enzyme solution described above, thereby yielding protoplasts.

Approximately equal numbers of iodoacetamide-treated tomato protoplasts and irradiated S. acaule protoplasts were mixed together and subjected to cell fusion according to the method of Kao et al., Planta 120: 215-27 (1974). The solutions for fusion treatment were modified as follows:

Five droplets of protoplast suspension solution were added, via a Pasteur pipette, to a plastic Petri dish; twelve minutes later, twelve droplets of PEG solution [25% (w/w) polyethylene glycol 1540, 10.5 mM $CaCl_2$ and 0.7 mM $KH_2PO_4$] were likewise added, and the solution was allowed to stand for twenty minutes. Twenty droplets of a high pH/high Ca solution comprised of 50.0 mM glycine-NaOH buffer (pH 10.5), 50.0 mM $CaCl_2$ and 0.2 M mannitol, as described by Keller and Melchers, Z. Naturforsch. 28: 737-41 (1973), were then added; after twenty minutes, twenty more droplets of the same high pH/high Ca solution were added after a comparable volume of liquid had first been removed from the suspension solution. Then an approximately twenty-droplet volume of the suspension solution was removed and a comparable volume added of a washing solution containing 0.75 mM $CaCl_2$ and 0.4 M mannitol. Fifteen minutes later, after a twenty- droplet volume of the suspension solution had been removed, an additional twenty droplets of the washing solution were added. After the washing solution was removed, twenty droplets of the TM2 solution described by the Shahin article were added and, fifteen minutes thereafter, the suspension solution was replaced with the same volume of TM2 medium.

Culturing and regeneration of the protoplast fusion products were carried out in accordance with the method described in the Shahin article. More specifically, two weeks after fusion the mini-calli obtained from the liquid medium were transferred onto a solid medium for a six-day culture at 24°C, under sixteen hours of light (500 lux). The calli which grew were transferred onto the regeneration medium described in the Shahin article for two-month culture at 24°C under sixteen hours of light (4500-5000 lux). Shoots which were generated under these conditions were transplanted onto TM-5 medium (see the Shahin article) for one-month culture. Shoots were rooted and aseptically propagated by cutting. About four months after fusion they were transferred to soil in a greenhouse.

Two plants regenerated from a callus ("Callus A"), obtained via fusing iodoacetamide-treated tomato protoplasts and S. acaule protoplasts irradiated with γ-rays (100 krad), had twenty-four chromosomes; the characters of these plants, including petal and anther characters, did not differ from those of the "Sekai-ichi" parent. Furthermore, the pollen of these plants, like "Sekai-ichi" pollen, stained almost 100% with acetocarmine.

But the pollen of these plants did not germinate at all on an artificial pollen-germination medium described by Brewbaker and Kwack, Amer. J. Botany 50: 859-65 (1963). This was in striking contrast to "Sekai-ichi" pollen, 80% of which germinated on this medium. The two regenerated plants failed to produce any seeds by self-pollination, and set seed only when they were pollinated with pollen from common tomato cultivars and Solanum pennellii, respectively. When pollinated with "Sekai-ichi" pollen, about 190 seeds were obtained per fruit (average of seven fruits).

When the hybrid seeds obtained by pollination with "Sekai-ichi" pollen were sown, the resulting plants did not differ from "Sekai-ichi" in morphological characteristics or in growth. Plants similarly regenerated from another callus ("Callus B"), which had been obtained in the same manner as Callus A except that x-rays were used instead of γ-rays, also had twenty-four chromosomes. Between 7% and 44% of the pollen of these plants stained with acetocarmine; the rest of the pollen grains were shrunken. The stainable pollen did not germinate at all on the above-mentioned artificial pollen germination medium.

No seeds were obtained from the Callus-B plants upon self-pollination, but seeds were obtained from all of the plants when they were pollinated with other fertile tomato cultivars, such as "Sekai-ichi."

Example 2.

The procedures of Example 1 were followed, except that each of the inbred tomato cultivars "Red Cherry," "VF-36", "Delicious" and "Kurihara" were used as a source of protoplasts, and 100 krad γ-ray-irradiated S. acaule leaves were the source for Solanum protoplasts.

The salient characteristics of each of the aforementioned cultivars are summarized below:

(1) Red Cherry - This is a cultivar of cherry tomato, and it was used as a representative cherry tomato in this experiment. The characteristics of "Red Cherry" are identical, except for fruit color, to those of the yellow cherry tomato. "Red Cherry" has been known for a very long time and was cultivated in Europe for many years. Although popular initially in home gardening, "Red Cherry" plants have recently been used extensively for the production of cherry tomatoes in Japan. Its maturity is intermediate between early-maturity and late-maturity varieties. Plants of this cultivar typically grow high, wide and straight. The stems and leaves are medium green and grow densely. The fruits grow in clusters of 7 to 9, and each fruit is small, round and red, with diameters of about 28 mm and weights of about nine grams. The fruit is divided into two chambers, each chamber containing pulp and small seeds. The taste of "Red Cherry" fruit is mild.

(2) VF-36 - An inbred developed at the University of California in 1959 from a cross of VC255 with VR11, VF-36 matures early and is resistant to Fusarium and Verticillium wilt. VF-36 has a determinate (self-topping) growth pattern, with fruits that are large and pointed in shape and that weigh between about 160 and 200 grams.

(3) Delicious - This cultivar has also been known for a long time, and there are many strains in existence. It matures early and is suitable for rapid cultivation. "Delicious" is suited to any kind of well-drained soil. It is resistant to overfertilization. The fruits are large, round and red, and have a very pleasing shape. Since the harvest period is very short, it is unreasonable to expect harvests to be large.

(4) Kurihara - An inbred line of long standing, "Kurihara" has vines that are vigorous, grow large and develop big leaves. It is suitable for open-field and slow cultivation, and thus is well-adapted to cultivation in greenhouses in the fall. It is suitable both as a slicing tomato and for a wide variety of processing applications. It is a high-yield plant which bears large-to-medium-sized fruits. The fruit is high-round but, when not enough fertilizer is added, tends to become angular. It can withstand a large amount of fertilizer, but fairly large harvests can be expected with just a small amount of fertilizer. Because of its soft skin, "Kurihara" fruit is not well-suited for transportation. The core is small. The very thin skin and soft pulp of "Kurihara" fruit means that the fruit cracks when overripe. Accordingly, careful timing of harvests is important.

Rooted shoots obtained from these cultivars were tested for pollen germination ability as shown in Table 1. According to the above-mentioned pollen germination criterion, all of the plants tested were completely male-sterile, with the exception of two plants, RC A-a and VF B-a (see Table 1).

One of the plants obtained from "Red Cherry" (RC B-b in Table 1) was not different from "Red Cherry" in terms of flower morphology. Also, pollen from the male-sterile plant stained with acetocarmine in the same manner as did parental "Red Cherry" pollen. But RC B-b pollen, unlike "Red Cherry" pollen, did not germinate on artificial pollen-germination medium. In addition, no seeds were obtained from the RC B-b plant after self-pollination. The plant set fruits about 24 mm in diameter (average of 9 fruits), and about 82 seeds were obtained per fruit upon pollination with pollen from "Red Cherry" (average of 4 fruits).

TABLE 1. Results of acetocarmine staining and pollen germination on artificial pollen germination medium

| cv.[1] | callus[2] | shoot | % pollen stained with acetocarmine | % germination |
|---|---|---|---|---|
| RC | A | a | 97 | 5 |
| | A | b | 63 | 0 |
| | A | c | 100 | 0 |
| | B | a | 100 | 0 |
| | B | b | 100 | 0 |
| | C | a | no pollen | - |
| | C | b | no pollen | - |
| | D | a | 64 | 0 |
| | D | b | 100 | 0 |
| VF | A | a | 87 | 0 |
| | A | b | 42 | 0 |
| | B | a | 42 | 9 |
| De | A | a | 45 | 0 |
| | B | a | 100 | 0 |
| | B | b | 100 | 0 |
| Ku | A | a | 22 | 0 |
| | B | a | no pollen | - |
| | C | a | 17 | 0 |
| | D | a | 63 | 0 |
| | D | b | 91 | 0 |
| | D | c | 61 | 0 |

[1] RC: "Red Cherry"    VF: "VF-36    De: "Delicious    Ku: Kurihara

[2] The upper-case alphabetical designations refer to different calli of the various cultivars. The lower designations refer to different plants derived from the various calli.

Male-sterile plants were also obtained when, instead of s. acaule, s. tuberosum was used as the source for solanum protoplasts, pursuant to the present invention.

The present invention makes possible the efficient conversion of any tomato cultivar to male-sterility within a short time, without producing any undesired, adverse affects on the resulting plants. The present

EP 0 363 819 B1

invention provides the means for producing a homogenous stand of male-sterile tomato plants, which is of great utility in obtaining tomato hybrids on a commercial basis. Also, male-sterile tomato plants produced by the method of the present invention have a seed-productive capacity that is practically equal to that of the parent plants and, in particular, are capable of producing seeds without any changes in the characteristics of the plant as the result of pollination with the pollen of the tomato plant used for fusion. In this regard, a male-sterile tomato plant of the present invention is quite different from male-sterile plants produced heretofore using conventional methods and, hence, represents a significant contribution to the production of F₁ tomato seeds and fruit.

**Claims**

1.  A method for the production of male-sterile tomato-plants, comprising the steps of
    treating tomato protoplasts with a cytoplasmic inactivator to produce tomato protoplasts that contain inactivated cytoplasmic elements; further comprising the step
    of producing Solanum protoplasts that contain inactivated nuclear elements, said step comprising an irradiation either of a Solanum plant or of Solanum protoplasts with a sterilizing dose of gamma-ray or x-ray radiation; followed by the steps of
    fusing said tomato protoplasts that contain inactivated cytoplasmic genetic material with said Solanum protoplasts that contain inactivated nuclear genetic material, to obtain a plurality of fusion products; and
    regenerating at least one fusion product of said plurality into a whole, male-sterile tomato plant.

2.  A method according to claim 1, wherein said Solanum protoplasts that contain inactivated nuclear elements are protoplasts of a potato plant or a plant of a potato-like Solanum species.

3.  A method according to claim 2, wherein said protoplasts are S. acaule protoplasts.

4.  A method according to claim 1, wherein said cytoplasmic inactivator is iodoacetate or a derivative thereof.

5.  A method according to claim 1, wherein said cytoplasmic inactivator is Rhodamine 6-G.

6.  A method according to claim 1, wherein said Solanum plant or Solanum protoplasts are irradiated at a dosage of gamma- or x-rays that is within the range of 10 to 200 krads.

**Patentansprüche**

1.  Verfahren zur Herstellung männlich-steriler Tomaten-pflanzen umfassend die Schritte:
    Behandeln von Tomatenprotoplasten mit einem cytoplasmatischen Inaktivator zur Herstellung von Tomatenprotoplasten, die inaktivierte cytoplasmatische Elemente enthalten; ferner umfassend den Schritt:
    der Herstellung von Solanum-Protoplasten, die inaktivierte nukleare Elemente enthalten, wobei der genannte Schritt eine Bestrahlung entweder einer Solanum-Pflanze oder von Solanum-Protoplasten mit einer sterilisierenden Dosis von Gamma-Strahlen oder Röntgenstrahlen aufweist; gefolgt von den Schritten:
    Fusionieren der genannten Tomatenprotoplasten, die inaktiviertes cytoplasmatisches genetisches Material enthalten, mit den genannten Solanum-Protoplasten, die inaktiviertes nukleares genetisches Material enthalten, um eine Vielzahl von Fusionsprodukten zu erhalten; und
    Regenerieren von mindestens einem Fusionsprodukt der genannten Vielzahl in eine ganze, männlichsterile Tomatenpflanze.

2.  Verfahren nach Anspruch 1, worin die genannten Solanum-Protoplasten, die inaktivierte nukleare Elemente enthalten, Protoplasten einer Kartoffelpflanze oder einer Pflanze von kartoffel-ähnlichen Solanum-Spezies sind.

3.  Verfahren nach Anspruch 2, worin die genannten Protoplasten S. acaule-Protoplasten sind.

9

**4.** Verfahren nach Anspruch 1, worin der genannte cytoplasmatische Inaktivator Iodacetat oder ein Derivat davon ist.

**5.** Verfahren nach Anspruch 1, worin der genannte cytoplasmatische Inaktivator Rhodamin 6-G ist.

**6.** Verfahren nach Anspruch 1, worin die genannte Solanum-Pflanze oder Solanum-Protoplasten mit einer Dosis von Gamma- oder Röntgenstrahlen bestrahlt werden, die im Bereich von 10 bis 200 krad liegt.

**Revendications**

**1.** Méthode de production de plants de tomate stériles mâle, comprenant les étapes consistant à :
- traiter des protoplastes de tomate avec un inactivateur cytoplasmique pour produire des protoplastes de tomate qui contiennent des éléments cytoplasmiques inactivés; le procédé comprenant en outre l'étape consistant à
- produire des protoplastes de Solanum qui contiennent des éléments nucléaires inactivés, ladite étape comprenant une irradiation d'un plant de Solanum ou de protoplastes de Solanum avec une dose stérilisante de rayons gamma ou de rayons X; ce qui est suivi par les étapes consistant à
- fusionner lesdits protoplastes de tomate, qui contiennent de la matière génétique cytoplasmique inactivée, avec lesdits protoplastes de Solanum qui contiennent de la matière génétique nucléaire inactivée, pour obtenir plusieurs produits de fusion; et
- régénérer au moins un produit de fusion de ce groupe pour former un plant entier de tomate stérile mâle.

**2.** Méthode selon la revendication 1, dans laquelle lesdits protoplastes de Solanum qui contiennent des éléments nucléaires inactivés sont des protoplastes d'un plant de pomme de terre ou d'un plant d'une espèce de Solanum analogue à de la pomme de terre.

**3.** Méthode selon la revendication 2, dans laquelle lesdits protoplastes sont des protoplastes de S.acaule.

**4.** Méthode selon la revendication 1, dans laqueue ledit inactivateur cytoplasmique est de l' iodoacétate ou un dérivé de ce composé.

**5.** Méthode selon la revendication 1, dans laquelle ledit inactivateur cytoplasmique est de la Rhodamine 6-G.

**6.** Méthode selon la revendication 1 dans laquelle ledit plant de Solanum ou les protoplastes de Solanum est ou sont irradié(s) à une dose de rayons gamma ou de rayons X qui se situe entre 10 et 200 krads.